Europäisches Patentamt

European Patent Office

Office européen des brevets

(11) Veröffentlichungsnummer: **0 416 604 A2**

# EUROPÄISCHE PATENTANMELDUNG

(21) Anmeldenummer: 90117148.8

(22) Anmeldetag: 06.09.90

(51) Int. Cl.⁵: **A61K 31/55**, A61K 31/34, A61K 31/44

(30) Priorität: 07.09.89 DE 3929763

(43) Veröffentlichungstag der Anmeldung:
13.03.91 Patentblatt 91/11

(84) Benannte Vertragsstaaten:
AT BE CH DE DK ES FR GB GR IT LI LU NL SE

(71) Anmelder: **Dr. Karl Thomae GmbH**
**Postfach 1755**
**W-7950 Biberach 1(DE)**

(72) Erfinder: **Van Meel, Jacques, Dr. Dipl.-Pharm.**
**Schubertweg 4**
**W-7951 Mittelbiberach(DE)**

(54) PAF-Antagonisten zur Herstellung eines Arzneimittels, welches zur Behandlung der durch verminderte beta-Rezeptorstimulation verursachten Herzkrankheiten geeignet ist.

(57) Die vorliegende Erfindung betrifft die neue Verwendung der PAF-Antagonisten zur Behandlung des durch verminderte $\beta$-Rezeptorstimulation am Herzen verursachten Krankheiten und deren Verwendung zur Herstellung eines entsprechenden Arzneimittels.

EP 0 416 604 A2

# PAF-ANTAGONISTEN ZUR HERSTELLUNG EINES ARZNEIMITTELS, WELCHES ZUR BEHANDLUNG DER DURCH VERMINDERTE β-REZEPTORSTIMULATION VERURSACHTEN HERZKRANKHEITEN GEEIGNET IST

PAF, der sogn. platelet activating factor, ist eine endogene Substanz, die bei verschiedenen pathophysiologischen Prozessen freigesetzt wird (siehe Braquet et al. in Pharmacol. Rev. 39 , 97-115 (1987)). Die wichtigsten hämodynamischen Effekte von PAF sind hierbei in der Blutdrucksenkung und in der Konstriktion der Koronargefäße zu sehen, außerdem wurde für PAF auch eine negativ inotrope Wirkung an Papillarmuskeln (siehe Robertson et al. in J. Pharmacol. Exp. Ther. 243 , 834-839 (1987)) beschrieben.

Zur Behandlung der durch PAF verursachten Krankheitsbilder eignen sich sogn. PAF-Antagonisten (siehe beispielsweise Prostaglandins 35 (5), 781-851 (1988)), also zur Behandlung und Vorbeugung von Entzündungen, Allergien, Asthma, Ödemen, bakteriellen Erkrankungen, Gefäßverengungen, insbesondere am Magen-Darmtrakt, des Gehirns und des Herzens, z.B. spastische Koronarverengungen, Akteriosklerose, arterielle und venöse Thrombosen sowie des anaphylaktischen und des Endotoxinschocks, insbesondere jedoch zur Behandlung der durch PAF induzierten cardiovasculären Erkrankungen, z.B. zur Behandlung des Mycocardinfarktes und zur Behandlung von Schockzuständen, also von akuten cardiovasculären Krankheitsbildern.

Außerdem ist bekannt, daß bei der chronischen Herzinsuffizienz die Ansprechbarkeit der cardialen β-Rezeptoren für Katecholamine stark vermindert ist (siehe Danielsen et al. in J. Cardiovasc. Pharmacol. 14 , 171-173 (1989)).

Es wurde nun gefunden, daß PAF nicht nur direkt negativ inotrop wirkt sondern zusätzlich selektiv die β-adrenergen Rezeptor-vermittelte Kontraktilität vermindert, was eine zusätzliche negative Komponente bei dem Krankheitsbild des Herzversagens und der Herzinsuffizienz darstellt.

PAF-Antagonisten eignen sich daher auch zur Behandlung der durch verminderte β-Rezeptorstimulation am Herzen bzw. durch Down Regulation (siehe Terrence P. Kenakin in "Pharmacologic Analysis of Drug-Receptor Interaction, Seiten 198-200", Raven Press, New York, 1987) der β-Rezeptoren am Herzen verursachten Krankheiten, z.B. zur Behandlung der zu geringen Pumpleistung des Herzens bei akutem Herzversagen, beispielsweise nach Myocardinfarkt und cardiogenem Schock, oder bei chronischen cardiovasculären Erkrankungen wie kongestive Herzinsuffizienz, Herzinsuffizienz nach Myocardinfarkt oder bei ischämischen Cardiomyopathien.

Gegenstand der vorliegenden Erfindung ist somit die neue Verwendung der PAF-Antagonisten zur Behandlung des durch verminderte β-Rezeptorstimulation am Herzen verursachten vorstehend erwähnten Krankheiten und deren Verwendung zur Herstellung eines entsprechenden Arzneimittels.

Erfindungsgemäß kommen hierbei beispielsweise die literaturbekannten PAF-Antagonisten, z. B. die aus der Tetrahydrofuran-, Thiazol-, Imido[2.1-a]isochinolin-, Dihydropyridin-, Thienodiazepin-, Benzodiazepin- oder Gingkolidreihe wie BN-50726, BN-50739, BN-52020 (Ginkgolid A), BN-52021 (Ginkgolid B), BN-52022 (Ginkgolid C), BN-52024 (Ginkgolid J), BN-52063, BN-52111, CV 6209, L 659886, L 659989, Ro 193704, Ro 244736, RP 48740, RP 59227, Sch 37370, SDZ-63-135, SDZ-63-441, SDZ-63-675, SDZ-64-412, WEB 2086, WEB 2098, WEB 2170 und Y 24180, in Betracht (siehe beispielsweise Pharmacol. Rev. 39 , 120-145 (1987), EP-A-0.176.928, EP-A-0.176.927, EP-A-0.176.929, EP-A-0.194.416, EP-A-0.230.942, EP-A-0.240.899, EP-A-0.254.245, EP-A-0.255.028, EP-A-0.268.242, EP-A-0.279.681, EP-A-0.284.359, EP-A-0.291.594, EP-A-0.298.466, EP-A-0.315.698, EP-A-0.316.456, EP-A-0.320.992, Br. J. Pharmac. 90 , 139-146 (1987), Blood and Vessel 16 , 558-572 (1985) und Drugs of the Future 11 869-875 (1986)), wobei die PAF-Antagonisten aus der Hetrazepinreihe (siehe Agents and Actions 27 , 473-476 (1989), EP-A-0.194.416, EP-A-0.230.942, EP-A-0.240.899, EP-A-0.254.245, EP-A-0.255.028, EP-A-0.268.242, EP-A-0.315.698, EP-A-0.316.456 und EP-A-0.320.992) bevorzugt sind, insbesondere aus der Thieno-[3.2-f][1.2.4]triazolo[4.3][1.4]diazepin- und 1.2.4-Triazolo[4.3-a][1.4]benzodiazepinreihe.

Beispielsweise seien folgende PAF-Antagonisten expressis verbis erwähnt:

cis-(±)-1-[2-[Hydroxy[[tetrahydro-5-[(octadecylaminocarbonyl)oxy]methyl]furan-2-yl]methoxyphosphinyloxy]-ethyl]chinoliniumhydroxid (SDZ-63441),

cis-(±)-1-[2-[Hydroxy[[tetrahydro-2,5-dimethyl-5-[(octadecylaminocarbonyl)oxy]methyl]furan-2-yl]-methoxyphosphinyloxy]ethyl]chinolinium-hydroxid (SDZ-63675),

trans-(±)-Tetrahydro-2-[3-methoxy-5-methylsulfonyl-4-propoxyphenyl]-5-(3,4,5-trimethoxyphenyl)-furan (L 659989),

(+)-N-[3-(Benzoyl)phenyl]-3-(3-pyridinyl)-1H,3H-pyrrolo-[1,2-c]thiazol-7-carboxyamid (RP 59227),

(+)-N-[3-(Benzyl)phenyl]-3-(3-pyridinyl)-1H,3H-pyrrolo[1,2-c]thiazol-7-carboxyamid,

3-(3-Pyridinyl)-1H,3H-pyrrolo[1,2-c]thiazol-7-carboxyamid (RP 4870),

2,3-Dihydro-5-[4-[2-(3,4,5-trimethoxyphenyl)ethyl]phenyl]imidazo[2,1-a]isochinolin (SDZ-64412)

EP 0 416 604 A2

5-(4-Trimethylsilylphenyl)-2,3-dihydro-imidazo[2,1-a]isochinolin (SDZ-63135),
1-O-(N-Stearylcarbamoyl)-2-O-(methoxycarbonyl)-3-O-[4-(thiazolium-3-yl)butyl]glycerol (RO 193704),
2-(2-Acetyl-6-methoxy-3,9-dioxo-4,8-dioxa-2,10-diazaoctacos-1-yl)-1-ethyl-pyridiniumchlorid (CV 6209),
3-tert.Butyl-hexahydro-4,7b,11-trihydroxy-8-methyl-9H-1,7a(epoxymethano)-1H,6aH-cyclopenta[c]furo[2,3-b]-
furo[3',2':3,4]cyclopenta[1,2-d]furan-5,9,12(4H)-trion (BN 52021),
BN-50726 (siehe Agents and Actions 27 473-476 (1989)),
4-(2-Chlorphenyl)-9-methyl-2-[2-[4-(2-methylpropyl)phenyl]ethyl]-6H-thieno[3,2-f][1,2,4]triazolo[4,3-a][1,4]-
diazepin (Y-24180),
1-Acetyl-4-(5,6-dihydro-8-methyl-11H-benzo[5,6]cyclohepta-[1,2-b]pyridin-11-yliden)-piperidin (Sch-37370),
1-[3-[4-(2-Chlorphenyl)-9-methyl-6H-thieno[3,2-f][1,2,4]triazolo[4,3-a][1,4]diazepin-2-yl]-2-propinyl]-2[1H]-
benzo[c]chinolinon (Ro 244736),
6-(2-Chlorphenyl)-8,9-dihydro-1-methyl-8-[(4-morpholinyl)-carbonyl]-4H,7H-cyclopenta[4.5]thieno[3,2-f]-
[1.2.4]triazolo-[4.3-a][1.4]diazepin (WEB 2170),
4-(2-Chlorphenyl)-9-methyl-2-[3-(4-morpholinyl)-3-propanon-1-yl]-6H-thieno[3.2-f][1.2.4]triazolo[4.3-a][1.4]-
diazepin (WEB 2086),
6-(2-Chlorphenyl)-8,9-dihydro-1-methyl-8-(dipropylaminocarbonyl)-4H,7H-cyclopenta[4,5]thieno[3,2-f][1,2,4]-
triazolo[4,3-a][1,4]diazepin (WEB 2347),
[4-(2-Chlorphenyl)-9-methyl-6H-thieno[3.2-f][1.2.4]triazolo-[4.3-a][1.4]diazepin-2-yl]-carbonsäuremorpholid,
[4-(2-Chlorphenyl)-9-methyl-6H-thieno[3.2-f][1.2.4]triazolo-[4.3-a][1.4]diazepin-2-yl]-carbonsäureamid,
2-[4-(2-Chlorphenyl)-9-methyl-6H-thieno[3.2-f][1.2.4]triazolo-[4.3-a][1.4]diazepin-2-yl]-ethan-1-
carbonsäurediethylamid, 2-[4-(2-Chlorphenyl)-9-methyl-6H-thieno[3.2-f][1.2.4]triazolo-[4.3-a][1.4]diazepin-2-
yl]-ethan-1-carbonsäure-N,N-di-(2-hydroxyethyl)amid],
2-[4-(2-Chlorphenyl)-9-methyl-6H-thieno[3.2-f][1.2.4]triazolo-[4.3-a][1.4]diazepin-2-yl]-ethan-1-
carbonsäuremethylamid,
2-[4-(2-Chlorphenyl)-9-methyl-6H-thieno[3.2-f][1.2.4]triazolo-[4.3-a][1.4]diazepin-2-yl]-ethan-1-
carbonsäureisopropylamid,
2-[4-(2-Chlorphenyl)-9-methyl-6H-thieno[3.2-f][1.2.4]triazolo-[4.3-a][1.4]diazepin-2-yl]-ethan-1-
carbonsäuredimethylamid,
2-[4-(2-Chlorphenyl)-9-methyl-6H-thieno[3.2-f][1.2.4]triazolo-[4.3-a][1.4]diazepin-2-yl]-ethan-1-carbonsäure-
N'-methyl-piperazid),
2-[4-(2-Chlorphenyl)-9-methyl-6H-thieno[3.2-f][1.2.4]triazolo-[4.3-a][1.4]diazepin-2-yl]-ethan-1-
carbonsäurepyrrolidid,
2-[4-(2-Chlorphenyl)-9-methyl-6H-thieno[3.2-f][1.2.4]triazolo-[4.3-a][1.4]diazepin-2-yl]-ethan-1-
carbonsäurepiperidid,
2-[4-(2-Chlorphenyl)-6H-thieno[3.2-f][1.2.4]triazolo[4.3-a][1.4]diazepin-2-yl]-ethan-1-carbonsäuremorpholid,
2-[4-(2-Chlorphenyl)-9-brom-6H-thieno[3.2-f][1.2.4]triazolo-[4.3-a][1.4]diazepin-2-yl]-ethan-1-
carbonsäuremorpholid,
2-[4-(2-Chlorphenyl)-9-methoxy-6H-thieno[3.2-f][1.2.4]triazo-[4.3-a][1.4]diazepin-2-yl]-ethan-1-
carbonsäuremorpholid,
4-(Morpholin-4-yl-carbonyl)-6-(2-chlorphenyl)-11-methyl-2,3,4,5-tetrahydro-8H-[1]benzothieno[3.2-f][1.2.4]-
triazolo-[4.3-a][1.4]diazepin,
3-(Morpholin-4-yl-carbonyl)-6-(2-chlorphenyl)-11-methyl-2,3,4,5-tetrahydro-8H-[1]benzothieno[3.2-f][1.2.4]-
triazolo-[4.3-a][1.4]diazepin,
3-(N,N-Diethylaminocarbonyl)-6-(2-chlorphenyl)-11-methyl-2,3,4,5-tetrahydro-8H-[1]benzothieno[3.2-f][1.2.4]-
triazolo-[4.3-a][1.4]diazepin,
4-(N,N-Diethylaminocarbonyl)-6-(2-chlorphenyl)-11-methyl-2,3,4,5-tetrahydro-8H-[1]benzothieno[3.2-f][1.2.4]-
triazolo-[4.3-a][1.4]diazepin,
4-(4-Methylpiperazinylcarbonyl)-6-(2-chlorphenyl)-11-methyl-2,3,4,5-tetrahydro-8H-[1]benzothieno[3.2-f]-
[1.2.4]triazolo-[4.3-a][1.4]diazepin,
4-(N-Methyl-N-2-hydroxyethylaminocarbonyl)-6-(2-chlorphenyl)-11-methyl-2,3,4,5-tetrahydro-8H-[1]-
benzothieno[3.2-f][1.2.4]triazolo[4.3-a][1.4]diazepin,
4-(N,N-Dimethylaminocarbonyl)-5-(2-chlorphenyl)-10-methyl-3,4-dihydro-2H,7H-cyclopenta[4.5]thieno[3.2-f]-
[1.2.4]triazolo-[4.3-a][1.4]diazepin,
3-(N,N-Diethylaminocarbonyl-5-(2-chlorphenyl)-10-methyl-3,4-dihydro-2H,7H-cyclopenta[4.5]thieno[3.2-f]-
[1.2.4]triazolo-[4.3-a][1.4]diazepin,
3-(N,N-Diethylaminocarbonyl-5-(2-chlorphenyl)-10-brom-3,4-dihydro-2H,7H-cyclopenta[4.5]thieno[3.2-f]-
[1.2.4]triazolo-[4.3-a][1.4]diazepin,
4-(Morpholin-4-yl-carbonylmethylaminocarbonyl-5-(2-chlorphenyl)-10-brom-3,4-dihydro-2H,7H-cyclopenta-

3

[4.5]thieno-[3.2-f][1.2.4]triazolo[4.3-a][1.4]diazepin,

4-(Morpholin-4-yl-carbonyl)-6-(2-chlorphenyl)-11-methyl-2,3,4,5-tetrahydro-8H-[1]benzothieno[3.2-f]imidazo-[1.2-a]-[1.4]diazepin,

4-(N,N-Diethylamino)-6-(2-chlorphenyl)-11-methyl-2,3,4,5-tetrahydro-8H-[1]benzothieno[1.2-a]-[1.4]diazepin,

3-(N-Morpholinomethyl)-5-(2-chlorphenyl)-10-methyl-3,4-dihydro-2H,7H-cyclopenta[4.5]thieno[3.2-f][1.2.4]-triazolo[4.3-a][1.4]diazepin,

3-(Acetoxymethyl)-5-(2-chlorphenyl)-10-methyl-3,4-dihydro2H,7H-cyclopenta[4.5]thieno[3.2-f][1.2.4]triazolo-[4.3-a][1.4]diazepin,

3-(1,2,4-Triazolyl-1-yl-methyl)-5-(2-chlorphenyl)-10-methyl-3,4-dihydro-2H,7H-cyclopenta[4.5]thieno[3.2-f]-[1.2.4]triazolo[4.3-a][1.4]diazepin,

3-(N-2,6-Dimethylmorpholino-methyl)-5-(2-chlorphenyl)-10-methyl-3,4-dihydro-2H,7H-cyclopenta[4.5]thieno-[3.2-f][1.2.4]triazolo[4.3-a][1.4]diazepin,

3-Acetoxymethyl-5-(2-chlorphenyl)-3,4-dihydro-2H,7H-cyclopenta[4.5]thieno[3.2-f][1.2.4]triazolo[4.3-a][1.4]-diazepin,

4-Acetoxymethyl-6-(2-chlorphenyl)-11-methyl-2,3,4,5-tetrahydro-8H-[1]benzothieno[3.2-f]imidazo[1.2-a][1.4]-diazepin,

3-Acetoxymethyl-5-(2-chlorphenyl)-10-methyl-3,4-dihydro-2H,7H-cyclopenta[4.5]thieno[3.2-f]imidazo[1.2-a]-[1.4]diazepin,

3-Diethylaminomethyl-5-(2-chlorphenyl)-10-methyl-3,4-dihydro-2H,7H-cyclopenta[4.5]thieno[3.2-f][1.2.4]-triazolo[4.3-a][1.4]diazepin,

4-Hydroxymethyl-6-(2-chlorphenyl)-11-methyl-2,3,4,5-tetrahydro-8H-[1]benzothieno[3.2-f][1.2.4]triazolo[4.3-a][1.4]diazepin,

4-(N-Morpholinomethyl)-6-(2-chlorphenyl)-11-methyl-2,3,4,5-tetrahydro-8H-[1]benzothieno[3.2-f][1.2.4]-triazolo[4.3-a][1.4]diazepin,

4-(Pyrazolyl-1-yl-methyl)-6-(2-chlorphenyl)-2,3,4,5-tetrahydro-8H-[1]benzothieno[3.2-f][1.2.4]triazolo[4.3-a]-[1.4]diazepin,

4-[4,4-Dimethyl-2-oxazolin-2-yl]-6-(2-chlorphenyl)-11-methyl2,3,4,5-tetrahydro-8H-[1]benzothieno[3.2-f]-[1.2.4]triazolo[4.3-a][1.4]diazepin,

4-[4,4-Dimethyl-2-imidazolin-2-yl]-6-(2-chlorphenyl)-11-methyl-2,3,4,5-tetrahydro-8H-[1]benzothieno[3.2-f]-[1.2.4]triazolo[4.3-a][1.4]diazepin,

4-[1,4,4-Trimethyl-imidazolin-2-yl]-6-(2-chlorphenyl)-11-methyl-2,3,4,5-tetrahydro-8H-[1]benzothieno[3.2-f]-[1.2.4]triazolo[4.3-a][1.4]diazepin,

4-Aminocarbonyl-6-(2-chlorphenyl)-11-methyl-2,3,4,5-tetrahydro-8H-[1]benzothieno[3.2-f][1.2.4]triazolo[4.3-a][1.4]diazepin,

4-[Morpholin-4-yl-carbonyl]-6-(2-chlorphenyl)-2,3,4,5-tetrahydro-8H-[1]benzothieno[3.2-f]imidazo[1.5-a][1.4]-diazepin,

4-[4,4-Dimethyl-2-thiazolin-2-yl]-6-(2-chlorphenyl)-11-methyl2,3,4,5-tetrahydro-8H-[1]benzothieno[3.2-f]-[1.2.4]triazolo[4.3-a][1.4]diazepin,

4-Amino-6-(2-chlorphenyl)-11-methyl-2,3,4,5-tetrahydro-8H-[1]benzothieno[3.2-f][1.2.4]triazolo[4.3-a][1.4]-diazepin,

3-(N-Morpholinyl-methyl)-5-(2-chlorphenyl)-10-methyl-3,4-dihydro-2H,7H-cyclopenta[4.5]thieno[3.2-f]imidazo-[1.2-a][1.4]diazepin,

4-(1,2,4-Triazol-1-yl-methyl)-6-(2-chlorphenyl)-11-methyl2,3,4,5-tetrahydro-8H-[1]benzothieno[3.2-f][1.2.4]-triazolo[4.3-a][1.4]diazepin und

4-(1,2,4-Triazol-1-yl-methyl)-6-(2-chlorphenyl)-11-methyl2,3,4,5-tetrahydro-8H-[1]benzothieno[3.2-f]imidazo-[1.2.-a][1.4]diazepin,

deren Enantiomeren und deren physiologisch verträglichen Säureadditionssalze.

Besonders wertvolle PAF-Antagonisten sind jedoch

trans-(±)-Tetrahydro-2-[3-methoxy-5-methylsulfonyl-4-propoxyphenyl]-5-(3,4,5-trimethoxyphenyl)-furan,

( + )-N-[3-(Benzoyl)phenyl]-3-(3-pyridinyl)-1H,3H-pyrrolo[1,2-c]thiazol-7-carboxyamid,

( + )-N-[3-(Benzyl)phenyl]-3-(3-pyridinyl)-1H,3H-pyrrolo[1,2-c]thiazol-7-carboxyamid,

3-(3-Pyridinyl)-1H,3H-pyrrolo[1,2-c]thiazol-7-carboxyamid,

2-(2-Acetyl-6-methoxy-3,9-dioxo-4,8-dioxa-2,10-diazaoctacos-1-yl)-1-ethyl-pyridiniumchlorid,

BN-50726 (siehe Agents and Actions 27, 473-476 (1989)),

4-(2-Chlorphenyl)-9-methyl-2-[2-[4-(2-methylpropyl)phenyl]ethyl]-6H-thieno[3,2-f][1,2,4]triazolo[4,3-a][1,4]-diazepin,

1-Acetyl-4-(5,6-dihydro-8-methyl-11H-benzo[5,6]cyclohepta[1,2-b]pyridin-11-yliden)-piperidin,

1-[3-[4-(2-Chlorphenyl)-9-methyl-6H-thieno[3,2-f][1,2,,4]triazolo[4,3-a][1,4]diazepin-2-yl]-2-propinyl]-2[1H]-benzo[c]chinolinon,

6-(2-Chlorphenyl)-8,9-dihydro-1-methyl-8-[(4-morpholinyl)-carbonyl]-4H,7H-cyclopenta[4.5]thieno[3,2-f]-[1.2.4]triazolo[4.3-a][1.4]diazepin,

4-(2-Chlorphenyl)-9-methyl-2-[3-(4-morpholinyl)-3-propanon1-yl]-6H-thieno[3.2-f][1.2.4]triazolo[4.3-a][1.4]-diazepin und

6-(2-Chlorphenyl)-8,9-dihydro-1-methyl-8-(dipropylaminocarbony    1)-4H,7H-cyclopenta[4,5]thieno[3,2-f]-[1,2,4]triazolo[4,3-a][1,4]diazepin,

deren Enantiomeren und deren physiologisch verträglichen Säureadditionssalze, insbesondere jedoch

(-)-6-(2-Chlorphenyl)-8,9-dihydro-1-methyl-8-[(4-morpholinyl)carbonyl]-4H,7H-cyclopenta[4.5]thieno[3,2-f]-[1.2.4]triazolo[4.3-a][1.4]diazepin (Substanz A),

4-(2-Chlorphenyl)-9-methyl-2-[3-(4-morpholinyl)-3-propanon-1-yl]-6H-thieno[3.2-f][1.2.4]triazolo[4.3-a][1.4]-diazepin (Substanz B) und

(2-Chlorphenyl)-8,9-dihydro-1-methyl-8-(dipropylaminocarbony  1,  4H,7H-cyclopenta[4,5]thieno[3,2-f][1,2,4]-triazolo[4,3-a][1,4]diazepin (Substanz C),

deren Enantiomeren und deren physiologisch verträglichen Säureadditionssalze.

Die neu aufgefundene Wirkung der PAF-Antagonisten wurde beispielsweise an

B  =  4-(2-Chlorphenyl)-9-methyl-2-[3-(4-morpholinyl)-3-propanon-1-yl]-6H-thieno[3.2-f][1.2.4]triazolo[4.3-a]-[1.4]diazepin

wie folgt geprüft:

Männliche Ratten (Chbb:THOM, ca. 300 g) werden mit Hexobarbital-Natrium (150 mg/kg i.p.) narkotisiert. Die Trachea wird kannuliert und danach werden die Tiere despinalisiert. Die Tiere werden künstlich beatmet und mittels einem Heizkissen auf 37° C gehalten. Zur Messung des linksventrikulärem Drucks wird über die Arteria Carotis in den linken Ventrikel ein Millar Tipmanometer geschoben. Dieses Signal wird elektronisch differentiert und ist als LV-dP/dtmax ein Maß für die Kontraktionskraft des Herzes. Über einen Katheter in der anderen Arteria Carotis wird der arterielle Blutdruck gemessen. Die zu untersuchenden Substanzen werden intravenös über eine Kanüle in der Vena jugularis injiziert.

Isoprenalin (3-100 ng/kg) steigert dosisabhänig LV-dP/dtmax von 300 ± 90 bis 4450 ± 290 mm Hg/s (n = 6). Nach einer 45-minütigen Infusion von PAF (50-100 ng/kg/min.) wurde wieder Isoprenalin (3 bis 100 ng/kg) injiziert. Die Effekte von 3 und 10 ng/kg Isoprenalin wurden hierdurch nicht beeinflußt, aber die Effekte auf LV-dP/dtmax von 30 und 100 ng/kg Isoprenalin waren stark vermindert (ca. 46 % Verminderung). Dann wurde die Substanz B (10 mg/kg i.v.) verabreicht und nach 10 Minuten wurde wieder Isoprenalin injiziert. Jetzt war die Ansprechbarkeit der cardialen β-Rezeptoren wieder voll da und die Steigerung von LV-dP/dtmax durch Isoprenalin war nicht signifikant unterschiedlich von den Kontrollwerten.

Ferner konnte auch gezeigt werden, daß PAF die positiv inotrope Wirkung von dem Histamin-2 Agonist Dimaprit (1-10 mg/kg) und dem Adenylcyclase Aktivator Forskolin (3-20 μg/kg) absolut nicht beeinflußt.

Auf Grund der neu aufgefundenen Wirkung der PAF-Antagonisten eignen sich diese auch zur Behandlung des durch verminderte β-Rezeptorstimulation am Herzen bzw. durch Down Regulation der β-Rezeptoren am Herzen verursachten Krankheiten, z.B. zur Behandlung der zu geringen Pumpleistung des Herzens bei akutem Herzversagen, beispielsweise nach Myocardinfarkt und cardiogenem Schock, oder bei chronischen cardiovasculären Erkrankungen wie kongestive Herzinsuffizienz, Herzinsuffizienz nach Myocardinfarkt oder bei ischämischen Cardiomyopathien, wie bereits eingangs erwähnt wurde.

Die akuten Toxizitäten werden in der Literatur von zahlreichen PAF-Antagonisten angegeben. Aus diesen Daten geht hervor, daß die PAF-Antagonisten gut verträglich und praktisch untoxisch sind, so weist beispielsweise die Substanz A eine von 1960 mg/kg/p.o., Substanz B eine $LD_{50}$ von 4600 mg/kg p.o. und Substanz C eine von 1700 mg/kg/p.o. jeweils an der Maus auf.

Die zur Erzielung der erfindungsgemäßen neuen Wirkung erforderliche Dosierung eines PAF-Antagonisten liegt hierbei im Bereich der bereits für PAF-Antagonisten angegebenen Dosierungen.

Bei oraler Dosierung liegt diese beim Erwachsenen zweckmäßigerweise zwischen 5 und 100, vorzugsweise zwischen 10 und 50 mg pro Dosis, und bei intravenösen oder intramuskulärer Application zwischen 1 und 75, vorzugsweise zwischen 1 und 25 mg pro Dosis, jeweils 1 bis 4 mal täglich, und bei Kindern je nach Lebensalter entsprechend niedriger, z. B. bei 1/4 bis 1/2 der vorstehend erwähnten Dosis.

Hierzu lassen sich die PAF-Antagonisten sowie ihre physiologisch verträglichen Additionssalze zusammen mit einem oder mehreren inerten Trägerstoffen in die üblichen galenischen Zübereitungen wie Tabletten, Dragées, Kapseln, Oblaten, Pulver, Suppositorien, Suspensionen oder Lösungen einarbeiten.

Die nachfolgenden Beispiele sollen die Erfindung näher erläutern ohne sie zu beschränken:

EP 0 416 604 A2

Beispiel 1

Tabletten, enthaltend 10 mg Substanz B

| Zusammensetzung: | |
|---|---|
| Substanz B | 10,0 mg |
| Maisstärke | 57,0 mg |
| Milchzucker | 48,0 mg |
| Polyvinylpyrrolidon | 4,0 mg |
| Magnesiumstearat | 1,0 mg |
| | 120,0 mg |

Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei ca. 45° C getrocknet. Das trockene Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten. Tablettengewicht: 120 mg

Beispiel 2

Dragées, enthaltend 5 mg Substanz B

| Zusammensetzung: | |
|---|---|
| Substanz B | 5,0 mg |
| Maisstärke | 41,5 mg |
| Milchzucker | 30,0 mg |
| Polyvinylpyrrolidon | 3,0 mg |
| Magnesiumstearat | 0,5 mg |
| | 80,0 mg |

Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45° C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.
Dragéegewicht: 130 mg

Beispiel 3

6

Tabletten, enthaltend 50 mg Substanz B

| Zusammensetzung: | |
|---|---|
| Substanz B | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:

Der Substanz B, Calciumphosphat, Milchzucker und Maisstärke werden mit einer wässrigen Polyvinylpyrrolidonlösung gleichmäßig befeuchtet. Die Masse wird durch ein 2 mm Sieb gegeben, im Umlufttrockenschrank bei 50 °C getrocknet und erneut gesiebt. Nach Zumischen des Schmiermittels wird das Granulat auf einer Tablettiermaschine gepreßt.

Beispiel 4

Kapseln, enthaltend 50 mg Substanz B

| Zusammensetzung: | |
|---|---|
| Substanz B | 50,0 mg |
| Maisstärke | 268,5 mg |
| Magnesiumstearat | 1,5 mg |
| | 320,0 mg |

Herstellung:

Substanz B und Maisstärke werden gemischt und mit Wasser befeuchtet. Die feuchte Masse wird gesiebt und getrocknet. Das trockene Granulat wird gesiebt und mit Magnesiumstearat gemischt. Die Endmischung wird in Hartgelatinekapseln Größe 1 abgefüllt.

Beispiel 5

Suppositorien, enthaltend 50 mg Substanz B

| Zusammensetzung: | |
|---|---|
| Substanz B | 50 mg |
| Adeps solidus | 1.650 mg |
| | 1.700 mg |

Herstellung:

Das Hartfett wird geschmolzen. Bei 40°C wird die gemahlene Wirksubstanz homogen dispergiert. Es wird auf 38°C abgekühlt und in schwach vorgekühlte Suppositorienformen ausgegossen.

Beispiel 6

Orale Suspension, enthaltend 50 mg Substanz B pro 5 ml

| Zusammensetzung: | |
| --- | --- |
| Substanz B | 50 mg |
| Hydroxyethylcellulose | 50 mg |
| Sorbinsäure | 5 mg |
| Sorbit 70%ig | 600 mg |
| Glycerin | 200 mg |
| Aroma | 15 mg |
| Wasser ad | 5 ml |

Herstellung:

Destilliertes Wasser wird auf 70°C erhitzt. Hierin wird unter Rühren Hydroxyethylcellulose gelöst. Durch Zugabe von Sorbitlösung und Glycerin wird auf Raumtemperatur abgekühlt. Bei Raumtemperatur werden Sorbinsäure, Aroma und Substanz B zugegeben. Zur Entlüftung der Suspension wird unter Rühren evakuiert.

Beispiel 7

Ampullen, enthaltend 1 mg Substanz B pro 5 ml

| Zusammensetzung: | |
| --- | --- |
| Substanz B | 1 mg |
| Natriumchlorid | 45 mg |
| Wasser für Injektionszwecke ad | 5 ml |

Herstellung:

Der Substanz B wird bei Eigen-pH in Wasser gelöst und Natriumchlorid als Isotonanz zugegeben. Die erhaltene Lösung wird pyrogenfrei filtriert und das Filtrat unter aseptischen Bedingungen in Ampullen abgefüllt, die anschließend sterilisiert und zugeschmolzen werden.

Beispiel 8

Ampullen, enthaltend 5 mg Substanz B pro 5 ml

| Zusammensetzung: | |
|---|---|
| Substanz B | 5 mg |
| Natriumchlorid | 45 mg |
| Wasser für Injektionszwecke ad | 5 ml |

Herstellung analog Beispiel 6.

Beispiel 9

Ampullen, enthaltend 10 mg Substanz B pro 5 ml

| Zusammensetzung: | |
|---|---|
| Substanz B | 10 mg |
| Methylglucamin | 35 mg |
| Glucofurol | 1000 mg |
| Polyethylenglykol-Polypropylenglykol-Blockpolymer | 250 mg |
| Wasser für Injektionszwecke ad | 5 ml |

Herstellung:

In einem Teil des Wassers wird Methylglucamin gelöst und der Substanz B unter Rühren in Lösung gebracht. Nach Zugabe der Lösungsmittel wird mit Wasser auf das Nennvolumen aufgefüllt.

Beispiel 10

Ampullen, enthaltend 20 mg Substanz B pro 10 ml

| Zusammensetzung: | |
|---|---|
| Substanz B | 20,000 mg |
| Natriumchlorid | 85,000 mg |
| Natriumdihydrogenphosphat x 2 $H_2O$ | 0,925 mg |
| Dinatriumhydrogenphosphat x 2 $H_2O$ | 1,320 mg |
| 0,1 NaOH | ad pH 6 |
| Wasser für Injektionszwecke | ad 10 ml |

Herstellung:

Die obigen Bestandteile werden in Wasser gelöst und nach Filtration wird die Lösung in sterilisierte braune Ampullen abgefüllt.

Beispiel 11

Inhalationslösung, enthaltend 5 mg Substanz B pro 1 ml

| Zusammensetzung: | |
| --- | --- |
| Substanz B | 500 mg |
| Na-EDTA | 50 mg |
| Benzalkoniumchlorid | 25 mg |
| Natriumchlorid | 880 mg |
| Destilliertes Wasser ad | 100 ml |

Herstellung:

96 % der Wassermenge werden vorgelegt, darin nacheinander Na-EDTA, Benzalkoniumchlorid, Natriumchlorid und Substanz B klar gelöst und mit dem restlichen Wasser aufgefüllt. Die Lösung wird in 20 ml Tropfflaschen abgefüllt.

Selbstverständlich lassen sich die übrigen erfindungsgemäßen PAF-Antagonisten in den für sie geeigneten Dosierungen in die üblichen galenischen Zubereitungen einarbeiten.

**Ansprüche**

1. Arzneimittel zur Behandlung der durch verminderte β-Rezeptorstimulation am Herzen bzw. durch Down Regulation der β-Rezeptoren am Herzen verursachten Krankheiten, enthaltend einen PAF-Antagonisten neben einem oder mehreren inerten Trägerstoffen.

2. Arzneimittel gemäß Anspruch 1, dadurch gekennzeichnet, daß dieses zur Behandlung der zu geringen Pumpleistung des Herzens bei akutem Herzversagen geeignet ist.

3. Arzneimittel gemäß Anspruch 1, dadurch gekennzeichnet, daß dieses zur Behandlung von chronischen cardiovasculären Erkrankungen wie kongestive Herzinsuffizienz, Herzinsuffizienz nach Myocardinfarkt oder von ischämischen Cardiomyopathien geeignet ist.

4. Arzneimittel nach mindestens einem der Ansprüche 1 bis 3, dadurch gekennzeichnet, daß dieses mindestens einen der PAF-Antagonisten aus der Reihe der Tetrahydrofuran-, Thiazol-, Imido[2.1-a]-isochinolin-, Dihydropyridin-, Thienodiazepin-, Benzodiazepin- oder Gingkolidderivate enthält.

5. Arzneimittel nach mindestens einem der Ansprüche 1 bis 3, dadurch gekennzeichnet, daß dieses mindestens einen der PAF-Antagonisten aus der Reihe der Hetrazepine enthält.

6. Arzneimittel nach mindestens einem der Ansprüche 1 bis 3, dadurch gekennzeichnet, daß dieses mindestens einen der nachfolgenden PAF-Antagonisten enthält:

trans-(±)-Tetrahydro-2-[3-methoxy-5-methylsulfonyl-4-propoxyphenyl]-5-(3,4,5-trimethoxyphenyl)-furan,

( + )-N-[3-(Benzoyl)phenyl]-3-(3-pyridinyl)-1H,3H-pyrrolo[1,2-c]thiazol-7-carboxyamid,

( + )-N-[3-(Benzyl)phenyl]-3-(3-pyridinyl)-1H,3H-pyrrolo[1,2-c]thiazol-7-carboxyamid,

3-(3-Pyridinyl)-1H,3H-pyrrolo[1,2-c]thiazol-7-carboxyamid, 2-(2-Acetyl-6-methoxy-3,9-dioxo-4,8-dioxa-2,10-diazaoctacos-1-yl)-1-ethyl-pyridiniumchlorid,

BN-50726 (siehe Agents and Actions 27 , 473-476 (1989)),

4-(2-Chlorphenyl)-9-methyl-2-[2-[4-(2-methylpropyl)phenyl]ethyl]-6H-thieno[3,2-f][1,2,4]triazolo[4,3-a][1,4]-diazepin,

1-Acetyl-4-(5,6-dihydro-8-methyl-IIH-benzo[5,6]cyclohepta[1,2-b]pyridin-11-yliden)-piperidin,

1-[3-[4-(2-Chlorphenyl)-9-methyl-6H-thieno[3,2-f][1,2,,4]triazolo[4,3-a][1,4]diazepin-2-yl]-2-propinyl]-2[1H]-benzo[c]chinolinon,

6-(2-Chlorphenyl)-8,9-dihydro-1-methyl-8-[(4-morpholinyl)-carbonyl]-4H,7H-cyclopenta[4.5]thieno[3,2-f]-[1.2.4]triazolo[4.3-a][1.4]diazepin,

4-(2-Chlorphenyl)-9-methyl-2-[3-(4-morpholinyl)-3-propanon1-yl]-6H-thieno[3.2-f][1.2.4]triazolo[4.3-a][1.4]-diazepin und

6-(2-Chlorphenyl)-8,9-dihydro-1-methyl-8-(dipropylaminocarbony        1)-4H,7H-cyclopenta[4,5]thieno[3,2-f]-

[1,2,4]triazolo[4,3-a][1,4]diazepin,
deren Enantiomeren und deren physiologisch verträglichen Säureadditionssalze.

7. Arzneimittel nach mindestens einem der Ansprüche 1 bis 3, dadurch gekennzeichnet, daß dieses mindestens einen der nachfolgenden PAF-Antagonisten enthält:

(-)-6-(2-Chlorphenyl)-8,9-dihydro-1-methyl-8-[(4-morpholinyl)carbonyl]-4H,7H-cyclopenta[4.5]thieno[3,2-f]-[1.2.4]triazolo[4.3-a][1.4]diazepin,

4-(2-Chlorphenyl)-9-methyl-2-[3-(4-morpholinyl)-3-propanon1-yl]-6H-thieno[3.2-f][1.2.4]triazolo[4.3-a][1.4]-diazepin und

6-(2-Chlorphenyl)-8,9-dihydro-1-methyl-8-(dipropylaminocarbony        1)-4H,7H-cyclopenta[4,5]thieno[3,2-f]-[1,2,4]triazolo[4,3-a][1,4]diazepin,
deren Enantiomeren und deren physiologisch verträglichen Säureadditionssalze.

8. Verwendung eines PAF-Antagonisten zur Herstellung eines Arzneimittels gemäß den Ansprüchen 1 bis 7, dadurch gekennzeichnet, daß dieses zur Behandlung der durch verminderte β-Rezeptorstimulation am Herzen bzw. durch Down Regulation der β-Rezeptoren am Herzen verursachten Krankheiten geeignet ist.